(19) Europäisches Patentamt

European Patent Office

Office européen des brevets

(11) **EP 4 541 289 A1**

(12) **EUROPEAN PATENT APPLICATION**

(43) Date of publication:
23.04.2025 Bulletin 2025/17

(21) Application number: 24207055.5

(22) Date of filing: 16.10.2024

(51) International Patent Classification (IPC):
*A61B 8/06* (2006.01)     *A61B 8/08* (2006.01)
*A61B 8/00* (2006.01)

(52) Cooperative Patent Classification (CPC):
A61B 8/06; A61B 8/0891; A61B 8/463;
A61B 8/488; A61B 8/5223

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 18.10.2023 JP 2023179561

(71) Applicant: FUJIFILM Corporation
Tokyo 106-8620 (JP)

(72) Inventor: FUJII, Nobuhiko
Tokyo (JP)

(74) Representative: Dehns Germany Partnerschaft
mbB
Theresienstraße 6-8
80333 München (DE)

(54) **ULTRASOUND DIAGNOSTIC APPARATUS**

(57) An object of the present invention is to set a display state of a Doppler waveform so that the Doppler waveform is easily visible.

An ultrasound diagnostic apparatus (100) includes a transmission unit (12), a reception unit (20), and an information processing unit (26). The transmission unit (12) transmits an ultrasonic wave to a subject via an ultrasound probe (14). The reception unit (20) receives the ultrasonic wave reflected by the subject (18) via the ultrasound probe (14). The information processing unit (26) executes processing on a reception signal output from the reception unit (20). The information processing unit (26) generates Doppler waveform data based on the reception signal, determines whether an observation site is an artery or a vein based on the Doppler waveform data, and decides a display range of blood flow velocity indicated by a Doppler waveform according to a determination result.

FIG. 1

**Description**

**BACKGROUND OF THE INVENTION**

1. Field of the Invention

The present disclosure relates to an ultrasound diagnostic apparatus, and particularly relates to processing for displaying a Doppler waveform.

2. Description of the Related Art

An ultrasound diagnostic apparatus that observes a subject by transmitting and receiving ultrasonic waves has been widely used. In general, the ultrasound diagnostic apparatus has a function of displaying a Doppler waveform indicating blood flow velocity. Waveform characteristic values such as a maximum value, a minimum value, and an average value of the Doppler waveform vary depending on an observation site. Therefore, a technique has been developed for adjusting a scale in displaying the Doppler waveform, thereby making it easier for a user to observe the Doppler waveform.

JP2010-88943A discloses that a Doppler velocity range is adjusted based on a statistical value of a distribution of at least one of maximum flow velocity or average flow velocity of a trace waveform of a spectrum signal in a frequency direction. In addition, JP2010-88943A discloses obtaining a baseline and multiples of how many times estimated values of an upper limit and a lower limit of the velocity range are set as a display range, based on a positive side maximum value and a negative side maximum value.

JP1999-235342A(JP-H11-235342A) discloses that, in an ultrasound color Doppler video system, it is determined whether a blood vessel as a diagnosis target is an artery or a vein based on a pulsation index PI and a resistance index RI of blood flow velocity. JP2016-87302A discloses a technique of generating Doppler image data representing blood flow velocity using color.

**SUMMARY OF THE INVENTION**

The waveform characteristic values of the Doppler waveform varies depending on the observation site. Therefore, in a case in which the Doppler waveform is displayed at a certain scale, it may become difficult to see the Doppler waveform.

An object of the present disclosure is to set a display state of a Doppler waveform so that the Doppler waveform is easily visible.

According to the present disclosure, there is provided an ultrasound diagnostic apparatus comprising: a transmission unit that transmits an ultrasonic wave to a subject via an ultrasound probe; a reception unit that receives the ultrasonic wave reflected by the subject via the ultrasound probe; and an information processing unit that executes processing on a reception signal output from the reception unit, in which the information processing unit generates Doppler waveform data based on the reception signal, determines whether an observation site is an artery or a vein based on the Doppler waveform data, and decides a display range of blood flow velocity indicated by a Doppler waveform according to a determination result.

In addition, according to the present disclosure, there is provided an ultrasound diagnostic apparatus comprising: a transmission unit that transmits an ultrasonic wave to a subject via an ultrasound probe; a reception unit that receives the ultrasonic wave reflected by the subj ect via the ultrasound probe; and an information processing unit that executes processing on a reception signal output from the reception unit, in which the information processing unit generates Doppler waveform data based on the reception signal, determines whether an observation site is an artery or a vein based on a degree of variation in a Doppler frequency of a plurality of the reception signals obtained through a plurality of times of transmission and reception of the ultrasonic wave, and decides a display range of blood flow velocity indicated by a Doppler waveform according to a determination result.

In addition, according to the present disclosure, there is provided an ultrasound diagnostic apparatus comprising: a transmission unit that transmits an ultrasonic wave to a subject via an ultrasound probe; a reception unit that receives the ultrasonic wave reflected by the subj ect via the ultrasound probe; and an information processing unit that executes processing on a reception signal output from the reception unit, in which the information processing unit generates Doppler waveform data and color Doppler data based on the reception signal, determines whether an observation site is an artery or a vein based on the color Doppler data, and decides a display range of blood flow velocity indicated by a Doppler waveform according to a determination result.

In one embodiment, the information processing unit determines whether the observation site is an artery or a vein based on whether an aliasing phenomenon occurs in the color Doppler data.

In one embodiment, the information processing unit decides a scale factor in displaying the Doppler waveform according to the determination result.

In one embodiment, the information processing unit decides the scale factor according to a Doppler waveform line obtained from the Doppler waveform data in addition to the determination result.

In one embodiment, the information processing unit obtains a reference value of a Doppler waveform line obtained from the Doppler waveform data, and decides the scale factor according to an area of a region between the Doppler waveform line obtained from the Doppler waveform data and a reference line indicated by the reference value in addition to the determination result.

In one embodiment, the information processing unit obtains a maximum value, a minimum value, and a reference value of a Doppler waveform line obtained from the Doppler waveform data, and decides the scale factor based on the maximum value, the minimum value, and the reference value in addition to the determination result.

In one embodiment, the information processing unit determines whether the observation site is an artery or a vein based on a time differential value of a Doppler waveform line obtained from the Doppler waveform data.

In one embodiment, the information processing unit determines whether the observation site is an artery or a vein based on a time integral value of a Doppler waveform line obtained from the Doppler waveform data.

In one embodiment, the information processing unit determines whether the observation site is an artery or a vein based on whether an aliasing phenomenon occurs in the Doppler waveform data.

In one embodiment, the ultrasound diagnostic apparatus further comprises: a pulsation detection device that detects pulsation of the subject, in which the information processing unit determines whether the observation site is an artery or a vein at a timing corresponding to the pulsation of the subject.

In one embodiment, a repetition frequency when the transmission unit transmits the ultrasonic wave is set according to the display range.

According to the present disclosure, it is possible to set a display state of a Doppler waveform so that the Doppler waveform is easily visible.

## BRIEF DESCRIPTION OF THE DRAWINGS

Fig. 1 is a diagram showing a configuration of an ultrasound diagnostic apparatus.

Fig. 2 is a diagram showing an example of a Doppler waveform.

Fig. 3 is a diagram showing an example of a B-mode color Doppler image and a Doppler waveform.

Fig. 4 is a diagram showing a Doppler waveform line together with a Doppler waveform.

Fig. 5 is a diagram showing a Doppler waveform line E(t) and polarity of a time differential value of the Doppler waveform line E(t).

Fig. 6 is a diagram showing a Doppler waveform line and an evaluation area.

Fig. 7 is a diagram showing an example of a B-mode color Doppler image and a Doppler waveform.

Fig. 8 is a diagram showing a Doppler waveform line together with a Doppler waveform.

Fig. 9 is a diagram showing an example of a B-mode color Doppler image and a Doppler waveform.

Fig. 10 is a diagram showing a Doppler waveform line together with a Doppler waveform.

Fig. 11 is a diagram showing an update timing of a scale factor.

Fig. 12 is a diagram showing an update timing of a scale factor.

## DESCRIPTION OF THE PREFERRED EMBODIMENTS

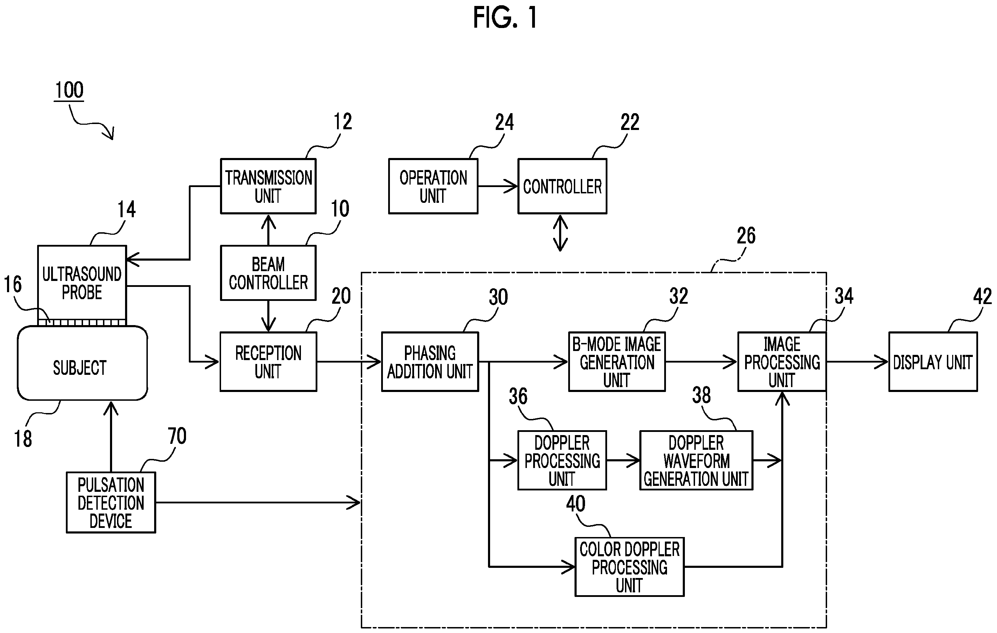

Each embodiment of the present disclosure will be described with reference to each drawing. The same components shown in a plurality of drawings are denoted by the same reference numerals, and the description thereof will not be repeated. Fig. 1 shows a configuration of an ultrasound diagnostic apparatus 100 according to an embodiment of the present disclosure. The ultrasound diagnostic apparatus 100 comprises a beam controller 10, a transmission unit 12, an ultrasound probe 14, a reception unit 20, a controller 22, an operation unit 24, an information processing unit 26, and a display unit 42.

The information processing unit 26 may be configured by one or a plurality of processors that execute a program to realize functions of a phasing addition unit 30, a B-mode image generation unit 32, an image processing unit 34, a Doppler processing unit 36, a Doppler waveform generation unit 38, and a color Doppler processing unit 40. The controller 22 performs overall control of the ultrasound diagnostic apparatus 100. The operation unit 24 includes a keyboard, a mouse, a lever, a button, a voice recognition device, and the like, and outputs information regarding a user's operation to the controller 22. The controller 22 controls the ultrasound diagnostic apparatus 100 in response to an operation on the operation unit 24.

The operation unit 24 may constitute a user interface for operating the ultrasound diagnostic apparatus 100 together with the display unit 42. For example, the operation unit 24 may include a touch panel on a display screen of the display unit 42. In addition, the operation unit 24 may have a function of operating a keyboard, a lever, a button, or the like that is virtually configured on the display screen. The controller 22 may output the information acquired via the user interface to the information processing unit 26.

An outline of an operation of the ultrasound diagnostic apparatus 100 will be described. The ultrasound diagnostic apparatus 100 transmits ultrasonic waves from the ultrasound probe 14 to a subject 18 and receives the ultrasonic waves reflected by the subject 18 via the ultrasound probe 14. The ultrasound diagnostic apparatus 100 executes measurements in B-mode, Doppler mode, and color Doppler mode.

In the B-mode operation, the ultrasound diagnostic apparatus 100 generates B-mode image data based on the ultrasonic waves received from the subject 18, and displays a B-mode image on the display unit 42. In the Doppler mode operation, the ultrasound diagnostic apparatus 100 generates Doppler waveform data regarding blood flow based on a Doppler shift frequency of the ultrasonic waves received from the subject 18, and displays the Doppler waveform on the display unit 42. In the color Doppler mode operation, the ultrasound diagnostic apparatus 100 generates image data showing an image in which a color corresponding to the blood flow velocity is applied to a B-mode image, and displays an image based on the image data on the display unit 42. Only one of the B-mode operation, the Doppler mode operation, or the color Doppler mode operation may be executed, or measurements in two or three of the three modes may be performed in a time division manner.

A specific configuration of the ultrasound diagnostic apparatus 100 and specific processing executed by the ultrasound diagnostic apparatus 100 will be described. The ultrasound probe 14 is in a state of being in contact with a surface of the subject 18. The ultrasound probe 14 comprises a plurality of transducer elements 16. The transmission unit 12 outputs a transmission signal to each of the transducer elements 16 of the ultrasound probe 14 under the control of the beam controller 10. As a result, the ultrasonic waves are transmitted from the ultrasound probe 14. The beam controller 10 controls the transmission unit 12 to form a transmission beam in the ultrasound probe 14 and scan the subject 18 with the transmission beam. That is, the transmission unit 12 adjusts a delay time or a level of each transmission signal in accordance with the control of the beam controller 10, forms a transmission beam in the ultrasound probe 14, and scans the subject 18 with the transmission beam.

In a case in which the ultrasonic waves reflected in the subject 18 are received by each transducer element 16 of the ultrasound probe 14, each transducer element 16 outputs an electric signal corresponding to the received ultrasonic waves to the reception unit 20. The reception unit 20 performs processing, such as amplification, detection, and frequency band limitation, on the reception signal output from each transducer element 16 in accordance with the control of the beam controller 10, and outputs the processed reception signal to the information processing unit 26. The information processing unit 26 performs processing on each reception signal, which is executed by each of the components (the phasing addition unit 30, the B-mode image generation unit 32, the image processing unit 34, the Doppler processing unit 36, the Doppler waveform generation unit 38, and the color Doppler processing unit 40).

The phasing addition unit 30 performs phasing addition of a plurality of the reception signals output from the reception unit 20 for the plurality of transducer elements 16 to generate a phase-adjusted reception signal. As a result, a phase-adjusted reception signal is generated through phase adjustment and addition such that the reception signals based on the ultrasonic waves received in a specific direction strengthen each other, and a reception beam is formed in the specific direction. The phase addition unit 30 outputs the phase-adjusted reception signal to the B-mode image generation unit 32 during the B-mode operation. In addition, the phase addition unit 30 outputs the phase-adjusted reception signal to the Doppler processing unit 36 during the Doppler mode operation and outputs the phase-adjusted reception signal to the color Doppler processing unit 40 during the color Doppler mode operation.

The B-mode operation will be described. The phasing addition unit 30 generates each phase-adjusted reception signal based on the ultrasonic wave received in each direction of the reception beam scanned in the subject 18, and outputs the phase-adjusted reception signal to the B-mode image generation unit 32. The B-mode image generation unit 32 generates B-mode image data based on the phase-adjusted reception signal obtained in each reception beam direction, and outputs the B-mode image data to the image processing unit 34. The B-mode image data based on one scan of the transmission beam and the reception beam (hereinafter, referred to as transmission and reception beams) is image data for one frame and corresponds to one B-mode image.

The beam controller 10, the transmission unit 12, the ultrasound probe 14, the reception unit 20, the phasing addition unit 30, and the B-mode image generation unit 32 generate the B-mode image data one after another in association with repetitive scan of the transmission and reception beams, and output each B-mode image data to the image processing unit 34. The image processing unit 34 generates a video signal for displaying the B-mode image based on the B-mode image data, and outputs the video signal to the display unit 42. The display unit 42 displays the B-mode image based on the video signal.

The Doppler mode operation will be described. The Doppler mode includes a pulse wave Doppler mode (PW Doppler mode) in which an ultrasonic pulse is transmitted at a repetition frequency PRF and a continuous wave Doppler mode (CW Doppler mode) in which the ultrasonic wave is continuously transmitted. Whether to operate in the PW Doppler mode or in the CW Doppler mode may be selected by an operation of the user.

The Doppler processing unit 36 sequentially performs fast Fourier transform processing on the phase-adjusted reception signal divided for a predetermined time length, sequentially generates frequency spectrum data of the Doppler shift frequency with an elapse of time, and outputs the frequency spectrum data to the Doppler waveform generation unit 38. Here, the Doppler shift frequency refers to a frequency representing a shift in frequency of the reception signal with respect to a frequency of the transmission signal.

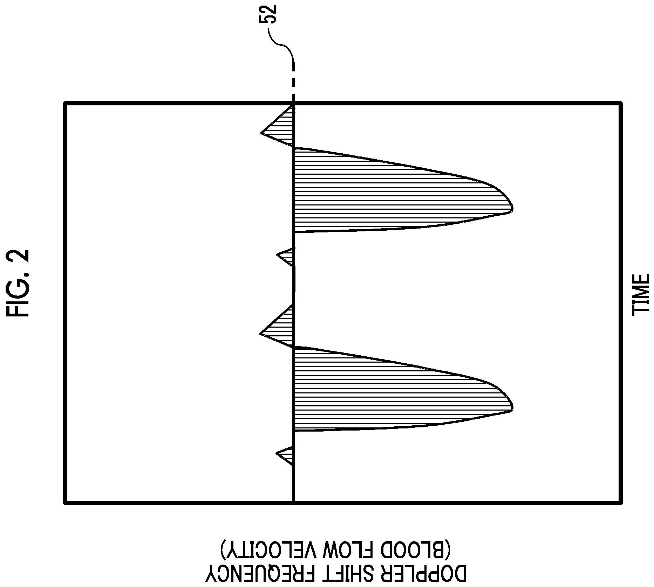

The Doppler waveform generation unit 38 generates Doppler waveform image data based on a plurality of sets of frequency spectrum data that are sequentially output with the elapse of time. Fig. 2 shows an example of the Doppler waveform indicated by the Doppler waveform data. In the Doppler waveform, a horizontal axis represents time and a vertical axis represents the Doppler shift frequency. The Doppler shift frequency shown on the vertical axis indicates blood flow velocity. Brightness of a plurality of images arranged in the vertical direction at a certain time point on the time axis indicates a frequency spectrum corresponding to the certain time point. A baseline 52 extending linearly in the horizontal direction indicates that the Doppler shift frequency, that is, the blood flow velocity is 0.

The Doppler waveform generation unit 38 sequentially updates the Doppler waveform data with the elapse of time. That is, the Doppler waveform generation unit 38 deletes the frequency spectrum data first obtained from the Doppler waveform data composed of a series of frequency spectrum data, and adds the latest frequency spectrum data to the Doppler waveform data to update the Doppler waveform data. The image processing unit 34 generates a video signal for displaying the Doppler waveform based on the Doppler waveform data and outputs the video signal to the display unit 42. The display unit 42 displays the Doppler waveform based on the video signal.

The color Doppler mode operation will be described. In a case in which the color Doppler processing unit 40 generates the color Doppler data, the ultrasonic waves are transmitted and received a plurality of J times in each direction of the transmission and reception beams. Here, J is an integer of 2 or more. The color Doppler processing unit 40 generates the color Doppler data based on the phase-adjusted reception signals acquired J times in each direction of the transmission and reception beams. The color Doppler data indicates blood flow velocity using color on a B-mode image of a region where scanning with the transmission and reception beams is performed. The color Doppler data may be data in which, for example, a blue color is applied to a region where the blood flows in a direction away from the ultrasound probe 14, a red color is applied to a region where the blood flows in a direction approaching the ultrasound probe 14, and brightness is increased in regions where the blood flow velocity is greater.

The color Doppler processing unit 40 obtains blood flow velocity v(r) in a depth direction of the transmission and reception beams based on the plurality of J phase-adjusted reception signals, for example, according to autocorrelation processing shown in JP2016-87302A. Here, v(r) indicates blood flow velocity at a position of depth r. The color Doppler processing unit 40 generates the color Doppler data based on the blood flow velocity v(r) in the depth direction at each depth r obtained in each direction of the transmission and reception beams, and outputs the color Doppler data to the image processing unit 34. Hereinafter, specific processing of obtaining the blood flow velocity v(r) will be described.

The phase-adjusted reception signal acquired at the q-th (q = 1 to J) of the first to J-th signals is represented by (Expression 1).

Expression 1

$$z(r, q) = I(r, q) + j \cdot Q(r, q)$$

r is a coordinate value in the depth direction. I(r,q) represents an in-phase component of the phase-adjusted reception signal, and Q(r,q) represents a quadrature component of the phase-adjusted reception signal, j is an imaginary unit. The color Doppler processing unit 40 obtains an autocorrelation value A(r) for J phase-adjusted reception signals z(r,1), z(r,2), ..., z(r,J) according to (Expression 2).

Expression 2

$$A(r) = \sum_{q=1}^{J-1} z(r, q) \cdot z(r, q + 1)^*$$

Here, the superscript "*" represents a complex conjugate. The color Doppler processing unit 40 obtains the blood flow velocity v(r) according to (Expression 3) using a real part Re[A(r)] and an imaginary part Im[A(r)] of the autocorrelation

value A(r).

Expression 3

$$v(r) = \frac{c}{2f_0} \cdot \frac{1}{2\pi T} arctan \left[ \frac{Im[A(r)]}{Re[A(r)]} \right]$$

c is propagation velocity of the ultrasonic wave propagating through the subject 18, and $f_0$ is a frequency of the ultrasonic wave transmitted and received by the ultrasound probe 14. A portion on a right side of (Expression 3) to the right of $c/(2f_0)$ represents a Doppler frequency. The blood flow velocity v(r) represents a component of the blood flow velocity in the depth direction at the depth r. In a case in which an angle φ formed by the blood flow direction with respect to the depth direction is known, v(r)/coscp is the blood flow velocity.

In a case in which it is not necessary to obtain an absolute value of the blood flow velocity (for example, a value in the MKSA unit system) such as in a case of measuring a blood flow velocity distribution, the blood flow velocity may be a value obtained by multiplying the value obtained in (Expression 3) by an optional constant. In addition, instead of the blood flow velocity, a Doppler shift frequency measurement value, which is the portion on the right side of (Expression 3) to the right of $c/(2f_0)$, may be used.

The image processing unit 34 generates data indicating a B-mode color Doppler image in which a color corresponding to the blood flow velocity is applied to the B-mode image, based on the B-mode image data and the color Doppler data. The image processing unit 34 generates a video signal for displaying the B-mode color Doppler image based on the B-mode color Doppler image data, and outputs the video signal to the display unit 42. The display unit 42 displays the B-mode color Doppler image based on the video signal.

The image processing unit 34 may display the B-mode image and the Doppler waveform side by side on the display unit 42. In addition, the image processing unit 34 may display the B-mode color Doppler image and the Doppler waveform side by side on the display unit 42.

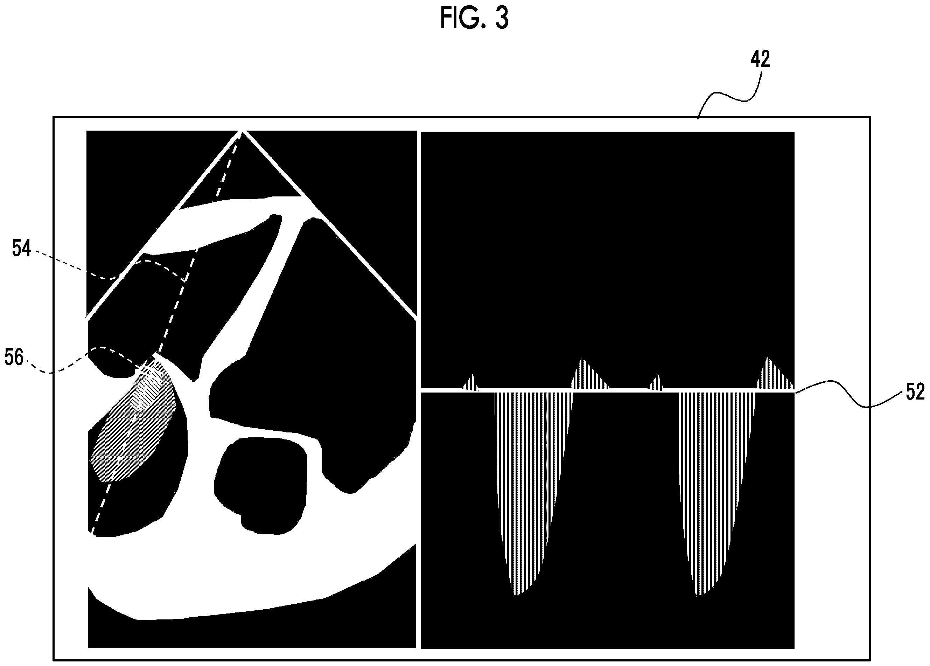

Fig. 3 shows an example of the B-mode color Doppler image and the Doppler waveform displayed on the display unit 42. The B-mode color Doppler image is shown on a left side, and the Doppler waveform is shown on a right side. Each image is acquired in the vicinity of a mitral valve of the heart. The shaded regions in the B-mode color Doppler image are colored regions.

On the B-mode color Doppler image, a beam straight line 54 indicating the direction of the transmission and reception beams in the Doppler mode and a Doppler gate 56 are shown. Here, the Doppler gate 56 refers to a range in which the blood flow velocity on the transmission and reception beams is measured and is set in an image of blood vessels appearing in the B-mode image. A region sandwiched between two parallel straight lines intersecting the beam straight line 54 is the Doppler gate 56, and the blood flow velocity is measured in this range. The controller 22 sets the beam straight line 54 and the Doppler gate 56 in response to the user's operation on the operation unit 24.

In a case in which the image processing unit 34 displays the Doppler waveform, the image processing unit 34 executes scaling. The scaling is a process of deciding a ratio of a swing width of the Doppler waveform to the display range of the display image in the vertical axis direction.

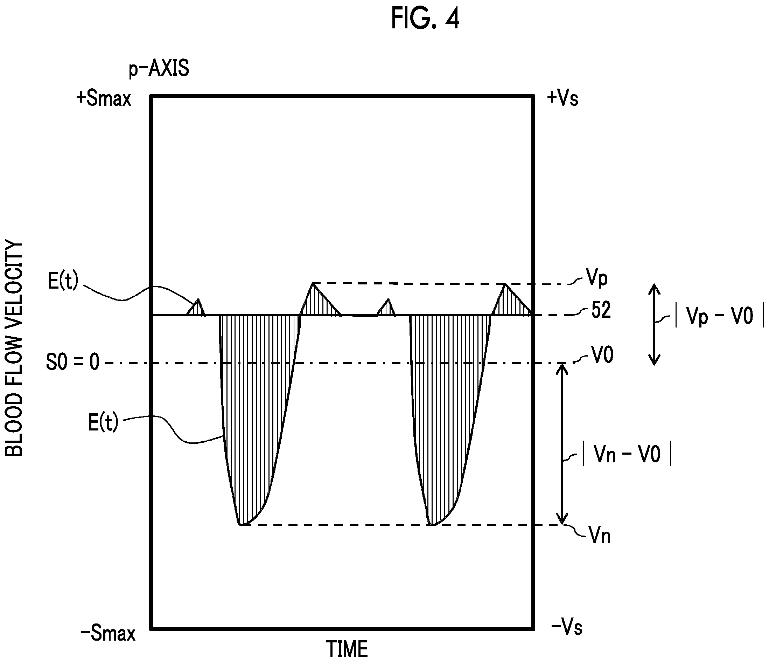

Fig. 4 shows a Doppler waveform for describing the scaling. A p-axis is defined as a vertical axis of an image coordinate system in the vertical direction of the display image. A coordinate value of the p-axis indicates a position in the vertical direction in the display image. In addition, a horizontal axis represents time. The image processing unit 34 obtains a display width Vs for each of the positive and negative sides based on the value indicated by the Doppler waveform, as described below. Further, the image processing unit 34 associates a positive value Vs of the display width of the Doppler waveform with an upper limit value of the p-axis in the image coordinate system, and associates a negative value -Vs of the display width of the Doppler waveform with a lower limit value of the p-axis. As a result, the display range of the blood flow velocity indicated by the Doppler waveform is decided to be -Vs or more and Vs or less.

The scaling executed by the image processing unit 34 will be specifically described. The image processing unit 34 obtains a Doppler waveform line E(t) based on the Doppler waveform data. The Doppler waveform line E(t) may be an envelope of the Doppler waveform. That is, the Doppler waveform line E(t) may be defined as a curve on a negative side of the Doppler waveform line E(t) in a positive region above the baseline 52, where a component value of the frequency spectrum corresponding to each time is equal to or greater than a predetermined threshold value. In addition, the Doppler waveform line E(t) may be defined as a curve on a positive side of the Doppler waveform line E(t) in a negative region below the baseline 52, where a component value of the frequency spectrum corresponding to each time is equal to or greater than a predetermined threshold value. Fig. 4 shows the Doppler waveform line E(t) together with the Doppler waveform.

The image processing unit 34 obtains, for example, a Doppler waveform average value in a time range traced back from the current point in time by predetermined N cardiac cycles, where N is a positive integer. The cardiac cycle may be measured by a pulsation signal output from a pulsation detection device 70 that detects the pulsation of the subject 18. The pulsation detection device 70 may be, for example, an electrocardiogram device. The pulsating signal may be a signal indicating an electrocardiogram. The Doppler waveform average value may be defined as a value obtained by averaging a midpoint value (half the value obtained by adding an upper limit value and a lower limit value) of the blood flow velocity range, where the component value of the frequency spectrum is equal to or greater than a predetermined threshold value, in a time range traced back from the current point in time by predetermined N cardiac cycles. The image processing unit 34 sets the Doppler waveform average value as a reference value V0. In addition, the image processing unit 34 obtains a maximum value Vp and a minimum value Vn of the Doppler waveform line E(t) in the time range traced back from the current point in time by the N cardiac cycles. The image processing unit 34 may obtain a maximum value Vp, a minimum value Vn, and a Doppler waveform average value in a time range traced back from the current point in time by a predetermined time.

The image processing unit 34 obtains a larger value of an absolute value $|Vp - V0|$ of a value obtained by subtracting the reference value V0 from the maximum value Vp, and an absolute value $|Vn - V0|$ of a value obtained by subtracting the reference value V0 from the minimum value Vn, as the swing width SW. That is, the image processing unit 34 obtains the swing width SW according to SW = max ($|Vp - V0|, |Vn - V0|$). Here, "max" indicates the maximum value of the values listed in the parentheses following "max".

The image processing unit 34 multiplies a predetermined scale factor k by the swing width SW to obtain the display width Vs as Vs = k · SW. The image processing unit 34 associates a positive value +Vs of the display width with an upper limit value Smax of the display range of the p-axis in the image coordinate system, and associates a negative value -Vs of the display width with a lower limit value -Smax of the display range of the p-axis in the image coordinate system. In addition, the image processing unit 34 associates the reference value V0 with a central value S0 of the display range of the p-axis.

The image processing unit 34 converts the value indicated by the Doppler waveform into the coordinate value of the p-axis such that an upper end of the display image indicates +Vs, a lower end of the display image indicates -Vs, and a central position of the display range in the vertical direction indicates the reference value V0. The image processing unit 34 generates a video signal for displaying the Doppler waveform based on the Doppler waveform data in which the value indicated by the Doppler waveform is converted into the coordinate value of the p-axis, and outputs the video signal to the display unit 42. The display unit 42 displays an image showing the Doppler waveform based on the video signal.

The scale factor k may be decided in advance based on a diagnosis result or the like performed in the past. The scale factor k may be read via the user interface. That is, the image processing unit 34 may acquire the scale factor k via the user interface and the controller 22. In a case of observing the vicinity of the mitral valve, the scale factor k may be, for example, 1.1 or more and less than 1.8.

In the example shown in Fig. 4, the swing width SW = max ($|Vp - V0|, |Vn - V0|$) is SW = $|Vn - V0|$, -Vs = $-|Vn - V0|$ is associated with the lower limit value of the p-axis, and +Vs = $|Vn - V0|$ is associated with the upper limit value of the p-axis.

The image processing unit 34 may execute a blood vessel determination to determine whether the observation site is an artery or a vein, and may decide the scale factor k according to the determination result. The image processing unit 34 in the present embodiment sets the scale factor k = ka in a case in which the observation site is determined to be an artery to be larger than the scale factor k = kv in a case in which the observation site is determined to be a vein.

In general, in a case in which a vein is observed, large noise appears in the image of the Doppler waveform. Therefore, in a case in which the scale factor k is increased, the noise is emphasized, and an image in which it is difficult to grasp the approximate shape of the Doppler waveform or the blood flow direction may be displayed. On the other hand, the Doppler waveform in a case in which an artery is observed has a large swing width, and the magnitude of the blood flow velocity indicated by the Doppler waveform is sufficiently large for the noise. In addition, since the shape of the Doppler waveform is generally observed in detail for the artery, increasing the scale factor k may make it easier to grasp the detailed shape of the Doppler waveform on the image.

In the ultrasound diagnostic apparatus 100 according to the present embodiment, the scale factor ka in a case in which the observation site is determined to be an artery is set to be larger than the scale factor kv in a case in which the observation site is determined to be a vein. Therefore, for the observation of the vein, an image in which the approximate shape of the Doppler waveform or the blood flow direction can be easily grasped is displayed, and for the observation of the artery, an image in which the detailed shape of the Doppler waveform can be easily grasped on the image is displayed.

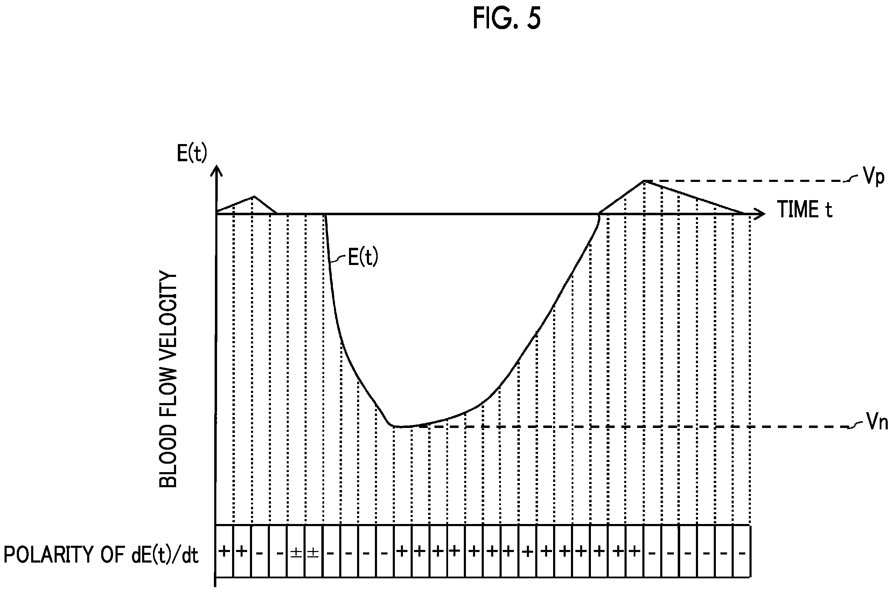

A specific example of the blood vessel determination executed by the image processing unit 34 will be described. The blood vessel determination may be performed based on polarity of a time differential value of the Doppler waveform. Fig. 5 shows the Doppler waveform line E(t) and polarity of a time differential value dE(t)/dt of the Doppler waveform line E(t). The image processing unit 34 stores a pattern in which polarity of a Doppler waveform line differential value changes with the elapse of time for each of an artery and a vein. This polarity change pattern may be a binary number in which a predetermined number of digits is assigned to L cardiac cycles. In addition, the polarity change pattern may be a polarity change pattern corresponding to a time slot from the first timing of the systole in the first cycle to the last timing of the diastole in the L-th cycle. Here, L is an optional positive integer. Further, the polarity change pattern may be a binary number in which 1 (high) is associated with positive polarity of the Doppler waveform line differential value and 0 (low) is associated with negative polarity of the Doppler waveform line differential value.

The image processing unit 34 obtains a degree of approximation between the polarity change pattern of the Doppler waveform line differential value and the polarity change pattern stored in advance for an artery. The degree of approximation may be, for example, a cross-correlation value. The image processing unit 34 determines that the observation site is an artery in a case in which the degree of approximation exceeds a predetermined threshold value Th1, and determines that the observation site is a vein in a case in which the degree of approximation is equal to or less than the threshold value Th1.

In addition, the image processing unit 34 may obtain a degree of approximation between the polarity change pattern of the Doppler waveform line differential value and the polarity change pattern stored in advance for a vein. In this case, the image processing unit 34 determines that the observation site is a vein in a case in which the degree of approximation exceeds a predetermined threshold value Th2, and the image processing unit 34 determines that the observation site is an artery in a case in which the degree of approximation is equal to or less than the threshold value Th2.

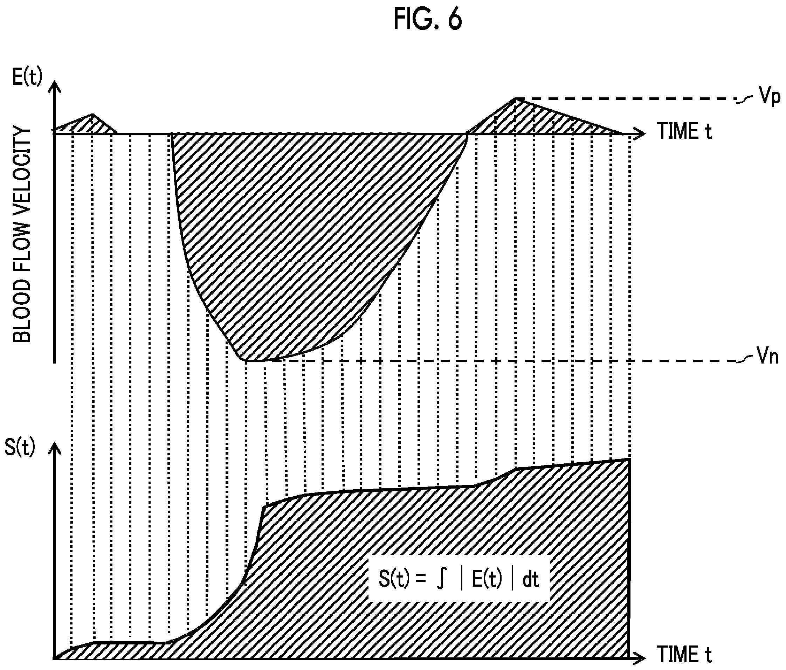

The blood vessel determination may be executed based on an evaluation area S(t) between the Doppler waveform E(t) and a reference line indicating the reference value V0. In Fig. 6, an upper part shows the Doppler waveform line E(t), and a lower part shows the evaluation area S(t).

The image processing unit 34 acquires the evaluation area S(t) at a predetermined time interval and obtains a time waveform of the evaluation area S(t) corresponding to the L cardiac cycles. The image processing unit 34 stores a time waveform pattern of the evaluation area S(t) for each of an artery and a vein. The time waveform pattern may be obtained by standardizing predetermined L cardiac cycles on a time axis with a predetermined time length.

The image processing unit 34 standardizes the time waveform of the evaluation area S(t) on the time axis with the predetermined L cardiac cycles and obtains a degree of approximation between the standardized time waveform and the time waveform pattern stored in advance for an artery. The degree of approximation may be, for example, a cross-correlation value. The image processing unit 34 determines that the observation site is an artery in a case in which the degree of approximation exceeds a predetermined threshold value Th3, and determines that the observation site is a vein in a case in which the degree of approximation is equal to or less than the threshold value Th3.

In addition, the image processing unit 34 may obtain a degree of similarity between the time waveform of the evaluation area S(t) standardized on the time axis and the time waveform pattern stored for a vein. In this case, in a case in which the degree of approximation exceeds a predetermined threshold value Th4, it is determined that the observation site is a vein, and in a case in which the degree of approximation is equal to or less than the threshold value Th4, it is determined that the observation site is an artery.

The image processing unit 34 may execute the blood vessel determination based on a degree of approximation between a Doppler waveform line E(t), which is a time differential value dS(t)/dt of the evaluation area S(t), and the time waveform pattern stored in advance for an artery or a vein, instead of the evaluation area S(t). In addition, the image processing unit 34 may execute the blood vessel determination based on a degree of approximation between a Doppler waveform line E(t), which is a second-order time differential value $d^2S(t)/dt^2$ of the evaluation area S(t), and the time waveform pattern stored in advance for an artery or a vein, instead of the evaluation area S(t).

In a case in which the image processing unit 34 is operated in the color Doppler mode, the blood vessel determination may be performed based on a degree of variation in the Doppler frequency of the phase-adjusted reception signals acquired a plurality of N times in a certain direction of the transmission and reception beams (hereinafter, referred to as a Doppler frequency variation degree). In a case in which the blood flow velocity v(r) is obtained according to (Expression 1) to (Expression 3), the Doppler frequency variation degree may be obtained based on J - 1 adjacent time Doppler frequencies obtained according to (Expression 4).

That is, the color Doppler processing unit 40 obtains the adjacent time Doppler frequency fa(r,q) for each of q = 1 to J - 1 according to (Expression 4) by using a real part and an imaginary part of z(r,q)·z(r,q + 1)* that are added together on a right side of (Expression 2).

Expression 4

$$fa(r, q) = \frac{1}{2\pi T} arctan \left[ \frac{Im[z(r, q) \cdot z(r, q + 1)^*]}{Re[z(r, q) \cdot z(r, q + 1)^*]} \right]$$

The color Doppler processing unit 40 obtains the Doppler frequency variation degree for J - 1 adjacent time Doppler frequencies fa(r,1), fa(r,2), ..., and fa(r,J - 1), and outputs the obtained Doppler frequency variation degree to the image processing unit 34. The Doppler frequency variation degree may be a value indicating variation such as variance and standard deviation for the J - 1 adjacent time Doppler frequencies.

The image processing unit 34 determines that the observation site is an artery in a case in which the Doppler frequency variation degree exceeds a predetermined threshold value Th5, and determines that the observation site is a vein in a case in which the Doppler frequency variation degree is equal to or less than the threshold value Th5.

In a case in which an aliasing phenomenon occurs while the ultrasound diagnostic apparatus 1 is operating in the CW Doppler mode or the color Doppler mode, the image processing unit 34 may determine that the blood vessel is an artery. Here, the aliasing phenomenon in the CW Doppler mode refers to phenomenon in which the Doppler waveform is periodically repeated and displayed in the vertical axis direction. The aliasing phenomenon in the color Doppler mode refers to a phenomenon in which the polarity of the blood flow velocity v(r) represented by (Expression 3) changes discontinuously with time change or region change. For example, there is a phenomenon in which the blood flow velocity v(r) increases in a negative direction with the elapse of time and then rapidly changes to a positive value. In addition, there is a phenomenon in which the blood flow velocity v(r) increases in a negative direction along a certain direction in a region where the blood flow velocity v(r) is acquired, and then rapidly changes to a positive value. In the B-mode color Doppler image, the aliasing phenomenon appears as a phenomenon in which a color changes rapidly with time change. In addition, in a case in which a blue color is applied to a region where the blood flows in a direction away from the ultrasound probe 14 and a red color is applied to a region where the blood flows in a direction approaching the ultrasound probe 14, the aliasing phenomenon appears as a phenomenon in which a blue color is applied to a region where a red color has to be applied or a red color is applied to a region where a blue color has to be applied.

In a case in which the aliasing phenomenon occurs, the controller 22 may adjust the repetition frequency PRF such that the aliasing phenomenon does not occur on the display image. In a case in which the scale factor k is increased, the controller 22 may control the beam controller 10, the transmission unit 12, the reception unit 20, and the information processing unit 26 such that the repetition frequency PRF is increased to a level at which the aliasing phenomenon is avoided. In addition, in a case in which the scale factor k is reduced, the controller 22 may control the beam controller 10, the transmission unit 12, the reception unit 20, and the information processing unit 26 such that the repetition frequency PRF is reduced. By reducing the repetition frequency PRF, a display resolution in the vertical axis direction is improved.

The image processing unit 34 may decide the scale factor ka for the artery and the scale factor kv for the vein based on an area between the Doppler waveform line E(t) and the reference line indicating the reference value V0. The image processing unit 34 obtains a time integral value of |Et(t) - V0|, for example, in a time range traced back from the current point in time by predetermined N cardiac cycles or in a time range traced back from the current point in time by a predetermined time. The image processing unit 34 may increase the scale factor ka for the artery and the scale factor kv for the vein as the time integral value decreases.

The image processing unit 34 may decide the scale factor ka for the artery such that a proportion R occupied by a region sandwiched between ka times the Doppler waveform line E(t) and the reference line in the display image is equal to or more than a certain threshold value Ta1 and equal to or less than a certain threshold value Ta2 (Ta1 ≤ R ≤ Ta2). For the vein, the scale factor kv may be decided such that a proportion R occupied by a region sandwiched between kv times the Doppler waveform line E(t) and the reference line in the display image is equal to or more than a certain threshold value Tv 1 and equal to or less than a certain threshold value Tv2 (Tv1 ≤ R ≤ Tv2). Here, the threshold value Ta1 is larger than the threshold value Tv2.

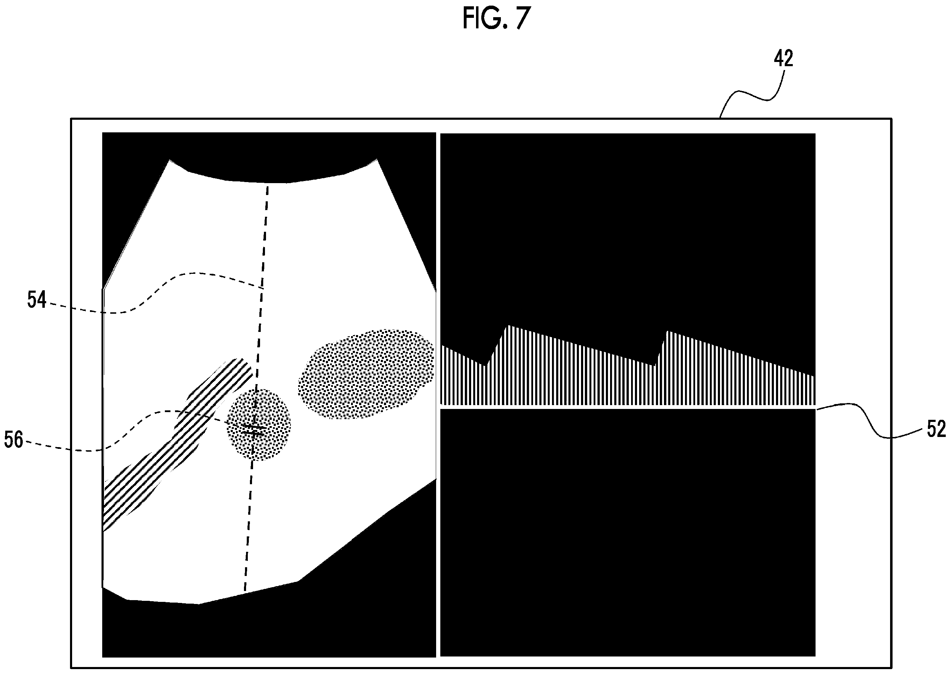

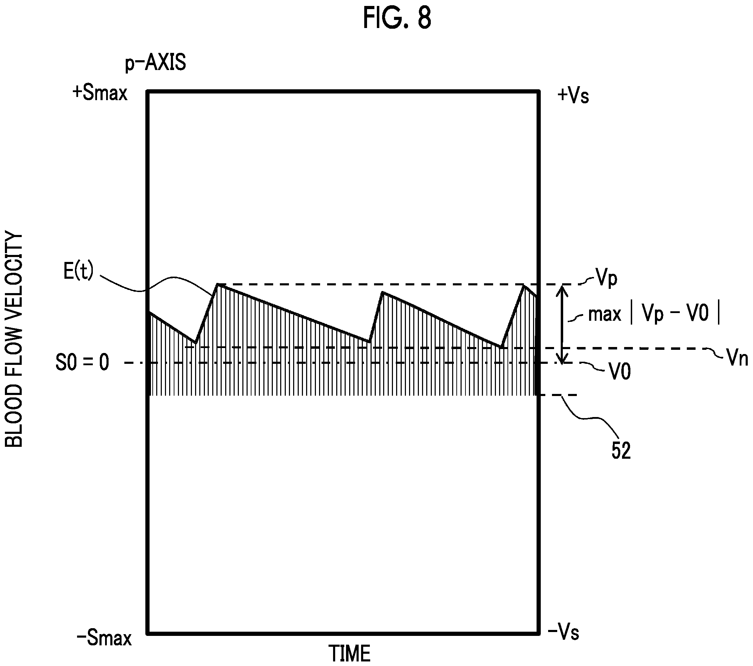

A case in which a part other than the vicinity of the mitral valve is observed will be described. An example of a B-mode color Doppler image acquired in the vicinity of the abdominal aorta is shown on a left side of Fig. 7. A Doppler waveform acquired in the vicinity of the abdominal aorta is shown on a right side of Fig. 7. In addition, Fig. 8 shows a Doppler waveform line E(t) together with the Doppler waveform. In the vicinity of the abdominal aorta, unlike the vicinity of the mitral valve of the heart, the direction of blood flow is unidirectional. In the example shown in Fig. 8, the reference value V0 is on a positive side with respect to the baseline 52, and the swing width SW is SW = max (|Vp - V0|,|Vn - V0|) = Vp - V0. The image processing unit 34 multiplies the scale factor k by the swing width SW to obtain the display width Vs as Vs = k · SW.

For the abdominal aorta, each threshold value or the like in the blood vessel determination is set such that the image processing unit 34 determines that the observation site is an artery. In a case of observing the vicinity of the abdominal aorta, the scale factor k may be, for example, 1.8 or more and less than 3.2.

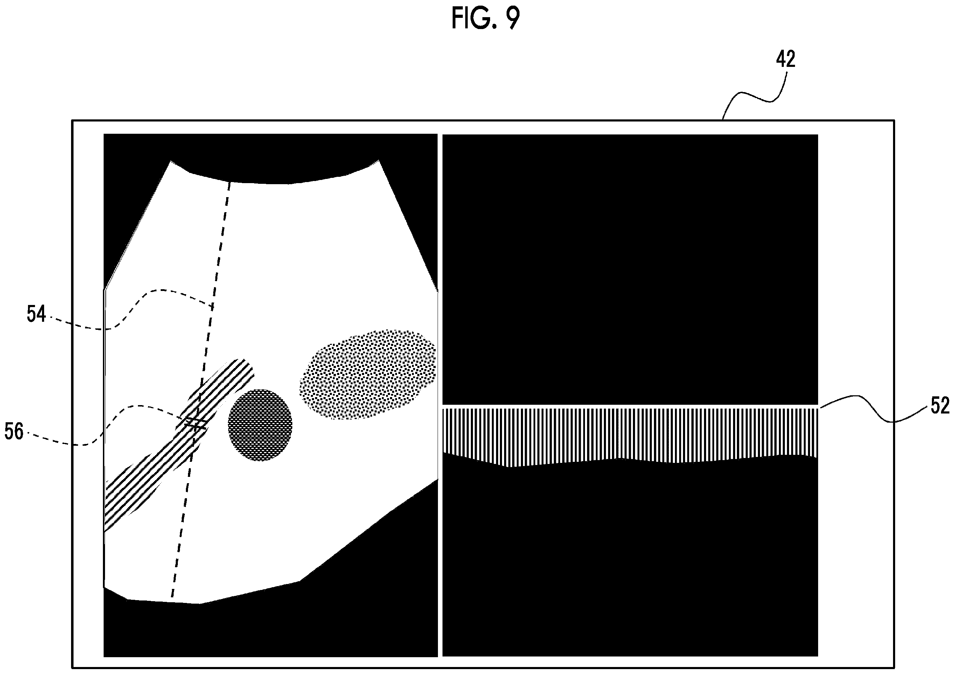

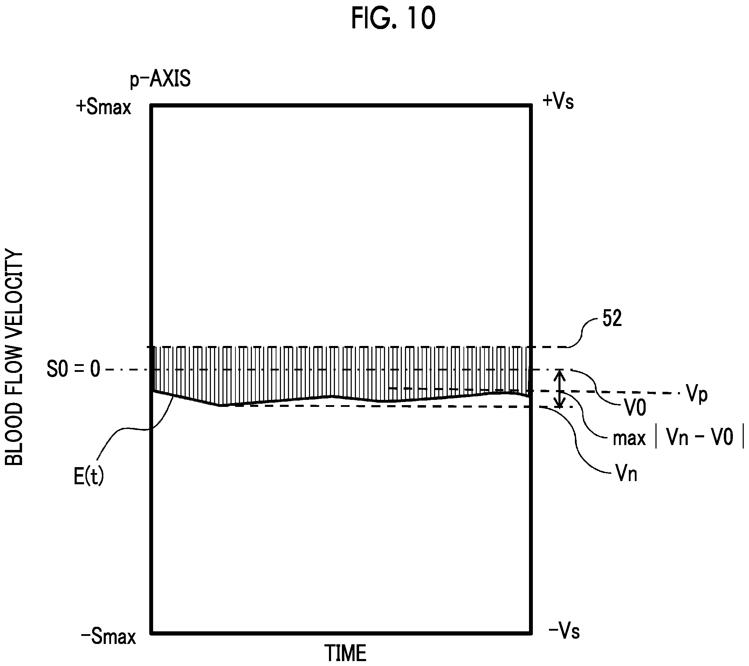

An example of a B-mode color Doppler image acquired in the vicinity of the portal vein of the liver is shown on a left side of Fig. 9. A Doppler waveform acquired in the vicinity of the portal vein is shown on a right side of Fig. 9. In addition, Fig. 10 shows a Doppler waveform line E(t) together with the Doppler waveform. In the vicinity of the portal vein, unlike the vicinity of the mitral valve of the heart, the direction of blood flow is unidirectional. In the example shown in Fig. 10, the reference value V0 is on a negative side with respect to the baseline 52, and the swing width SW is SW = max (|Vp - V0|,|Vn - V0|) = V0 - Vn. The image processing unit 34 multiplies the scale factor k by the swing width SW to obtain the display width Vs as Vs = k · SW.

For the portal vein, each threshold value or the like in the blood vessel determination may be set such that the image processing unit 34 determines that the observation site is an artery, or each threshold value or the like in the blood vessel determination may be set such that the image processing unit 34 determines that the observation site is a vein. In a case of observing the vicinity of the portal vein, the scale factor k may be, for example, 2.4 or more and less than 9.0.

In the above description, an embodiment is shown in which the reference value V0 is the Doppler waveform average value. The reference value V0 may be 0 or may be the centroid of the Doppler waveform. The centroid of the Doppler waveform is defined, for example, as a value obtained by calculating a weighted average value of the blood flow velocity using the component values of the frequency spectrum as weighting values for each time point, and further averaging these weighted average values of the blood flow velocity in a time range traced back from the current point in time by predetermined N cardiac cycles. In addition, the centroid of the Doppler waveform may be defined as a value obtained by averaging the weighted average values of the blood flow velocity in a time range traced back from the current point in time by a predetermined time.

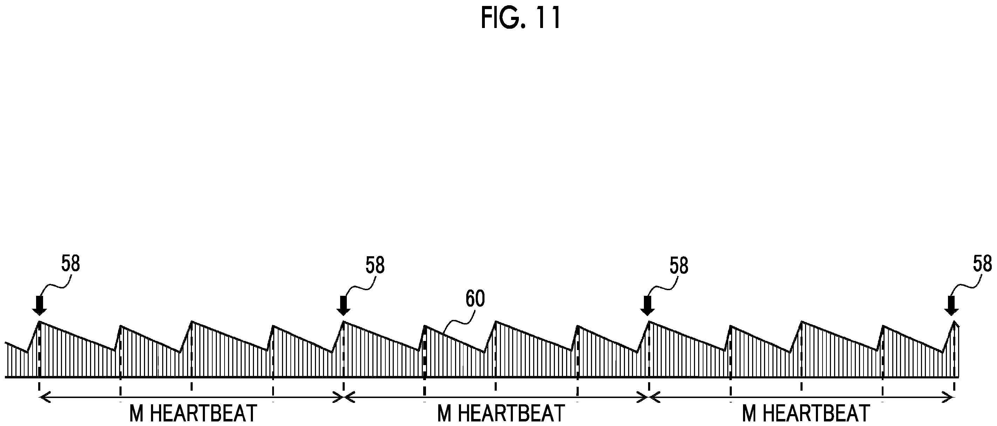

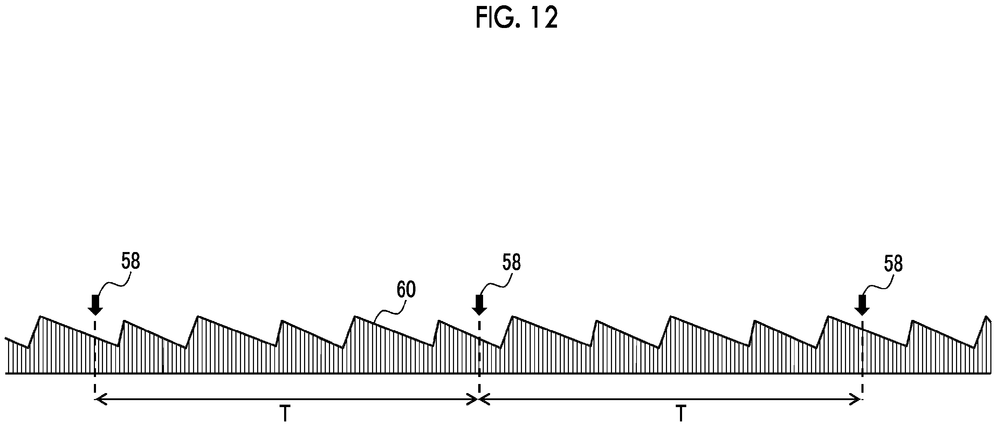

The image processing unit 34 may execute the blood vessel determination at a timing corresponding to the pulsation of the subject 18 and update the scale factor k. For example, the image processing unit 34 may execute the blood vessel determination for each M cardiac cycle, where M is a positive integer, and update the scale factor k. Fig. 11 conceptually shows an update timing 58 of the scale factor k and the Doppler waveform 60 in a case in which the blood vessel determination is performed for each M cardiac cycle and the scale factor k is updated. In addition, the image processing unit 34 may execute the blood vessel determination for each update cycle T, where T is an update cycle, and update the scale factor k. Fig. 12 conceptually shows an update timing 58 of the scale factor k and the Doppler waveform 60 in a case in which the blood vessel determination is performed for each update cycle T and the scale factor k is updated.

In addition, in a case in which an operation for updating the scale factor k is performed by the operation unit 24, the image processing unit 34 may execute the blood vessel determination and update the scale factor k.

The image processing unit 34 may automatically execute the blood vessel determination and set the scale factor k according to a diagnosis status. The diagnosis status is specified by, for example, an observation site such as the heart, liver, or abdomen, and a lesion that may be found in each observation site. The image processing unit 34 may recognize the diagnosis status via the user interface.

In addition, the image processing unit 34 may sequentially obtain the swing width SW = max ($|Vp - V0|,|Vn - V0|$) in a time range traced back from the current point in time by predetermined N cardiac cycles or in a time range traced back from the current point in time by a predetermined time. In a case in which the subsequently obtained swing width SW changes beyond a predetermined threshold value ThSW with respect to the previously obtained swing width SW, the image processing unit 34 may automatically execute the blood vessel determination and set the scale factor k.

Configuration of Present Disclosure

Configuration 1:

An ultrasound diagnostic apparatus comprising:

a transmission unit that transmits an ultrasonic wave to a subject via an ultrasound probe;
a reception unit that receives the ultrasonic wave reflected by the subject via the ultrasound probe; and
an information processing unit that executes processing on a reception signal output from the reception unit,
in which the information processing unit

generates Doppler waveform data based on the reception signal,
determines whether an observation site is an artery or a vein based on the Doppler waveform data, and
decides a display range of blood flow velocity indicated by a Doppler waveform according to a determination result.

Configuration 2:

An ultrasound diagnostic apparatus comprising:

a transmission unit that transmits an ultrasonic wave to a subject via an ultrasound probe;
a reception unit that receives the ultrasonic wave reflected by the subject via the ultrasound probe; and
an information processing unit that executes processing on a reception signal output from the reception unit,
in which the information processing unit

generates Doppler waveform data based on the reception signal,
determines whether an observation site is an artery or a vein based on a degree of variation in a Doppler frequency of a plurality of the reception signals obtained through a plurality of times of transmission and reception of the ultrasonic wave, and
decides a display range of blood flow velocity indicated by a Doppler waveform according to a determination result.

Configuration 3:

An ultrasound diagnostic apparatus comprising:

a transmission unit that transmits an ultrasonic wave to a subject via an ultrasound probe;
a reception unit that receives the ultrasonic wave reflected by the subject via the ultrasound probe; and
an information processing unit that executes processing on a reception signal output from the reception unit,
in which the information processing unit

generates Doppler waveform data and color Doppler data based on the reception signal,
determines whether an observation site is an artery or a vein based on the color Doppler data, and
decides a display range of blood flow velocity indicated by a Doppler waveform according to a determination result.

Configuration 4:

The ultrasound diagnostic apparatus according to Configuration 3,
in which the information processing unit determines whether the observation site is an artery or a vein based on whether an aliasing phenomenon occurs in the color Doppler data.

Configuration 5:

The ultrasound diagnostic apparatus according to any one of Configuration 1 to Configuration 4,
in which the information processing unit decides a scale factor in displaying the Doppler waveform according to the determination result.

Configuration 6:

The ultrasound diagnostic apparatus (100) according to claim 5,
wherein the information processing unit (26) decides the scale factor according to a Doppler waveform line obtained from the Doppler waveform data in addition to the determination result.

Configuration 7:

The ultrasound diagnostic apparatus according to Configuration 6,
in which the information processing unit

obtains a reference value of a Doppler waveform line obtained from the Doppler waveform data, and
decides the scale factor according to an area of a region between the Doppler waveform line obtained from the Doppler waveform data and a reference line indicated by the reference value in addition to the determination result.

Configuration 8:

The ultrasound diagnostic apparatus according to Configuration 6,
in which the information processing unit

obtains a maximum value, a minimum value, and a reference value of a Doppler waveform line obtained from the Doppler waveform data, and
decides the scale factor based on the maximum value, the minimum value, and the reference value in addition to the determination result.

Configuration 9:

The ultrasound diagnostic apparatus according to Configuration 1,

in which the information processing unit determines whether the observation site is an artery or a vein based on a time differential value of a Doppler waveform line obtained from the Doppler waveform data.

Configuration 10:

The ultrasound diagnostic apparatus according to Configuration 1,
in which the information processing unit determines whether the observation site is an artery or a vein based on a time integral value of a Doppler waveform line obtained from the Doppler waveform data.

Configuration 11:

The ultrasound diagnostic apparatus according to Configuration 1,
in which the information processing unit determines whether the observation site is an artery or a vein based on whether an aliasing phenomenon occurs in the Doppler waveform data.

Configuration 12:

The ultrasound diagnostic apparatus according to any one of Configuration 1 to Configuration 11, further comprising:

a pulsation detection device that detects pulsation of the subject,
in which the information processing unit determines whether the observation site is an artery or a vein at a timing corresponding to the pulsation of the subject.

Configuration 13:

The ultrasound diagnostic apparatus according to any one of Configuration 1 to Configuration 12,
in which a repetition frequency when the transmission unit transmits the ultrasonic wave is set according to the display range.

Explanation of References

10: beam controller
12: transmission unit
14: ultrasound probe
16: transducer element
18: subject
20: reception unit
22: controller
24: operation unit
26: information processing unit
30: phasing addition unit
32: B-mode image generation unit
34: image processing unit
36: Doppler processing unit
38: Doppler waveform generation unit
40: color Doppler processing unit
42: display unit
52: baseline
54: beam straight line
56: Doppler gate
58: update timing
60: Doppler waveform
70: pulsation detection device
100: ultrasound diagnostic apparatus

**Claims**

1. An ultrasound diagnostic apparatus (100) comprising:

   a transmission unit (12) configured to transmit an ultrasonic wave to a subject (18) via an ultrasound probe (14);
   a reception unit (20) configured to receive the ultrasonic wave reflected by the subject (18) via the ultrasound probe (14); and
   an information processing unit (26) configured to execute processing on a reception signal output from the reception unit (20),
   wherein the information processing unit (26) is configured to:

   generate Doppler waveform data based on the reception signal,
   determine whether an observation site is an artery or a vein based on the Doppler waveform data, and
   decide a display range of blood flow velocity indicated by a Doppler waveform according to a determination result.

2. An ultrasound diagnostic apparatus (100) comprising:

   a transmission unit (12) configured to transmit an ultrasonic wave to a subject (18) via an ultrasound probe (14);
   a reception unit (20) configured to receive the ultrasonic wave reflected by the subject (18) via the ultrasound probe (14); and
   an information processing unit (26) configured to execute processing on a reception signal output from the reception unit (20),
   wherein the information processing unit (26) is configured to:

   generate Doppler waveform data based on the reception signal,
   determine whether an observation site is an artery or a vein based on a degree of variation in a Doppler frequency of a plurality of the reception signals obtained through a plurality of times of transmission and reception of the ultrasonic wave, and
   decide a display range of blood flow velocity indicated by a Doppler waveform according to a determination result.

3. An ultrasound diagnostic apparatus (100) comprising:

   a transmission unit (12) configured to transmit an ultrasonic wave to a subject (18) via an ultrasound probe (14);
   a reception unit (20) configured to receive the ultrasonic wave reflected by the subject (18) via the ultrasound probe (14); and
   an information processing unit (26) configured to execute processing on a reception signal output from the reception unit (20),
   wherein the information processing unit (26) is configured to:

   generate Doppler waveform data and color Doppler data based on the reception signal,
   determine whether an observation site is an artery or a vein based on the color Doppler data, and
   decide a display range of blood flow velocity indicated by a Doppler waveform according to a determination result.

4. The ultrasound diagnostic apparatus (100) according to claim 3,
   wherein the information processing unit (26) is configured to determine whether the observation site is an artery or a vein based on whether an aliasing phenomenon occurs in the color Doppler data.

5. The ultrasound diagnostic apparatus (100) according to any one of claims 1 to 4,
   wherein the information processing unit (26) is configured to decide a scale factor (k, ka, kv) in displaying the Doppler waveform according to the determination result.

6. The ultrasound diagnostic apparatus (100) according to claim 5,
   wherein the information processing unit (26) is configured to decide the scale factor (k, ka, kv) according to a Doppler waveform line (E(t)) obtained from the Doppler waveform data in addition to the determination result.

7. The ultrasound diagnostic apparatus (100) according to claim 6,
   wherein the information processing unit (26) is configured to:

   obtain a reference value (V0) of a Doppler waveform line (E(t)) obtained from the Doppler waveform data, and decide the scale factor (k, ka, kv) according to an area (S(t)) of a region between the Doppler waveform line obtained from the Doppler waveform data and a reference line indicated by the reference value in addition to the determination result.

8. The ultrasound diagnostic apparatus (100) according to claim 6 or claim 7,
   wherein the information processing unit (26) is configured to:

   obtain a maximum value ($V_p$), a minimum value ($V_n$), and a reference value ($V_0$) of the Doppler waveform line (E(t)) obtained from the Doppler waveform data, and
   decide the scale factor (k, ka, kv) based on the maximum value, the minimum value, and the reference value in addition to the determination result.

9. The ultrasound diagnostic apparatus (100) according to any preceding claim,
   wherein the information processing unit (26) is configured to determine whether the observation site is an artery or a vein based on a time differential value (dE(t)/dt) of a Doppler waveform line (E(t)) obtained from the Doppler waveform data.

10. The ultrasound diagnostic apparatus (100) according to any preceding claim,
    wherein the information processing unit (26) is configured to determine whether the observation site is an artery or a vein based on a time integral value (|Et(t) - V0|) of a Doppler waveform line (E(t)) obtained from the Doppler waveform data.

11. The ultrasound diagnostic apparatus (100) according to any preceding claim,
    wherein the information processing unit (26) is configured to determine whether the observation site is an artery or a vein based on whether an aliasing phenomenon occurs in the Doppler waveform data.

12. The ultrasound diagnostic apparatus (100) according to any preceding claim, further comprising:

    a pulsation detection device (70) configured to detect pulsation of the subject,
    wherein the information processing unit (26) is configured to determine whether the observation site is an artery or a vein at a timing corresponding to the pulsation of the subject.

13. The ultrasound diagnostic apparatus (100) according to any preceding claim,
    wherein a repetition frequency (PRF) at which the transmission unit transmits the ultrasonic wave is set according to the display range.

## FIG. 1

EP 4 541 289 A1

## FIG. 2

FIG. 3

# FIG. 4

p-AXIS

+Smax / +Vs

BLOOD FLOW VELOCITY

E(t) → Vp

52

$|Vp - V0|$

S0 = 0 ····· V0

E(t)

$|Vn - V0|$

Vn

-Smax / -Vs

TIME

EP 4 541 289 A1

FIG. 5

E(t)

Vp

TIME t

E(t)

Vn

BLOOD FLOW VELOCITY

POLARITY OF dE(t)/dt

| + | + | - | - | ± | - | - | - | + | + | + | + | + | + | + | + | + | + | - | - | - | - | - | - |

FIG. 6

$$S(t) = \int | E(t) | \, dt$$

EP 4 541 289 A1

FIG. 7

# FIG. 8

EP 4 541 289 A1

FIG. 9

FIG. 10

EP 4 541 289 A1

FIG. 11

EP 4 541 289 A1

FIG. 12

Europäisches
Patentamt

European
Patent Office

Office européen
des brevets

# EUROPEAN SEARCH REPORT

Application Number

EP 24 20 7055

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/058471 A1 (KONINKL PHILIPS ELECTRONICS NV [NL]; BHATTACHARYA PURANJOY [IN] ET AL.) 19 May 2011 (2011-05-19) | 1,2,9, 10,12 | INV. A61B8/06 A61B8/08 |
| Y | * page 7, line 19 - line 25 * <br> * page 10, line 20 * <br> * figures 1,2,7 * <br> - - - - - | 5-8 | A61B8/00 |
| X | US 2020/229795 A1 (TADROSS RIMON [US] ET AL) 23 July 2020 (2020-07-23) <br> * paragraphs [0025], [0034]; figure 2 * <br> - - - - - | 3,4,11, 13 | |
| Y | US 2020/405270 A1 (OTA KAZUSHI [JP] ET AL) 31 December 2020 (2020-12-31) <br> * paragraph [0122]; figure 9 * <br> - - - - - | 5-8 | |

**TECHNICAL FIELDS SEARCHED (IPC)**

A61B

The present search report has been drawn up for all claims

| Place of search | Date of completion of the search | Examiner |
|---|---|---|
| Munich | 31 January 2025 | Marzal-Abarca, X |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 7055

This annex lists the patent family members relating to the patent documents cited in the above-mentioned European search report.
The members are as contained in the European Patent Office EDP file on
The European Patent Office is in no way liable for these particulars which are merely given for the purpose of information.

31-01-2025

| Patent document cited in search report | | Publication date | Patent family member(s) | | Publication date |
|---|---|---|---|---|---|
| WO 2011058471 | A1 | 19-05-2011 | NONE | | |
| US 2020229795 | A1 | 23-07-2020 | NONE | | |
| US 2020405270 | A1 | 31-12-2020 | JP | 7334494 B2 | 29-08-2023 |
| | | | JP | 2021003214 A | 14-01-2021 |
| | | | US | 2020405270 A1 | 31-12-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010088943 A
- JP 11235342 A
- JP H11235342 A
- JP 2016087302 A